# EUROPEAN PATENT SPECIFICATION

(11) **EP 2 524 693 B1**
(45) Date of publication and mention of the grant of the patent: **21.05.2014**
(21) Application number: 11732936.7
(22) Date of filing: 13.01.2011
(51) Int. Cl.: A61K 31/4188, A61K 31/7088, A61K 39/395, A61K 45/00, A61K 48/00, A61P 9/00, A61P 27/02, A61P 27/06, A61P 35/00, A61P 43/00

(54) **PHARMACEUTICAL FOR PREVENTING OR TREATING DISORDERS ACCOMPANIED BY OCULAR ANGIOGENESIS AND/OR ELEVATED OCULAR VASCULAR PERMEABILITY**
PHARMAZEUTIKUM ZUR VERHINDERUNG ODER BEHANDLUNG VON DURCH OKULARE ANGIOGENESE UND/ODER ERHÖHTE AUGENGEFÄSSDURCHLÄSSIGKEIT BEGLEITETEN ERKRANKUNGEN
PRODUIT PHARMACEUTIQUE DESTINÉ AU TRAITEMENT PROPHYLACTIQUE OU THÉRAPEUTIQUE DE TROUBLES ACCOMPAGNÉS D'UNE ANGIOGENÈSE OCULAIRE ET/OU D'UNE PERMÉABILITÉ VASCULAIRE OCULAIRE SUPÉRIEURE À LA NORMALE

(30) Priority: 12.03.2010 JP 2010055479; 14.01.2010 JP 2010006159
(43) Date of publication of application: 21.11.2012
(73) Proprietor: Sanwa Kagaku Kenkyusho Co., Ltd, Nagoya-shi, Aichi 461-8631 (JP); Nagoya City University, Nagoya-shi, Aichi 467-8601 (JP)
(72) Inventor: OGURA Yuichiro, Nagoya-shi Aichi 467-8601 (JP); NOZAKI Miho, Nagoya-shi Aichi 467-8601 (JP); NAKAJIMA Atsushi, Nagoya-shi Aichi 461-8631 (JP); HIBI Chihiro, Nagoya-shi Aichi 461-8631 (JP)
(74) Representative: Duckworth, Timothy John
(86) International application number: PCT/JP2011/050472
(87) International publication number: WO 2011/087066

(56) References cited:
- EP-A1- 1 723 956
- EP-A1- 2 110 141
- EP-A2- 1 719 774
- WO-A2-2008/063932
- WO-A2-2010/009034
- JP-A- 7 242 547
- JP-A- 2005 511 576
- SIMO R ET AL: "Intravitreous anti-VEGF for diabetic retinopathy: hopes and fears for a new therapeutic strategy", DIABETOLOGIA, vol. 51, no. 9, September 2008 (2008-09), pages 1574-1580, XP002695272, ISSN: 0012-186X
- KASTELAN SNJEZANA ET AL: "PHARMACOTHERAPY FOR DIABETIC RETINOPATHY - IT IS NOT JUST A DREAM", DIABETOLOGIA CROATICA, vol. 37, no. 3, 2008, pages 57-66, XP002695273, ISSN: 0351-0042
- JOURNAL OF DIABETES AND ITS COMPLICATIONS vol. 17, 2003, pages 374 - 379
- DIABETES vol. 52, March 2003, pages 864 - 871, XP002990017

## Description

### Technical Field

The present invention relates to a pharmaceutical for use in preventing or treating a disorder accompanied by ocular angiogenesis and/or increased ocular vascular permeability, which is composed of a combination of an anti-VEGF agent and a specific hydantoin derivative.

### Background Art

Vascular endothelial growth factor (VEGF) is the most responsible for angiogenesis. Examples of anti-VEGF agents may include receptor antagonists, anti-VEGF antibodies, VEGF Ligand inhibitors, and oligonucleotide drugs, relating to VEGF as angiogenetic factors. Specific examples include bevacizumab sodium, sorafenib, sunitinib, pegaptanib sodium, ranibizumab, aflibercept, and VEGF-Trap EYE. These are used as intravitreal injections in the field of ophthalmology. The major pharmacological actions of these anti-VEGF agents are suppression of angiogenesis, suppression of vascular permeability and suppression of the proliferation of vascular endothelial cells.

It has been clarified in recent years that VEGF is involved in various ocular diseases such as age-related macular degeneration (AMD), based on their physiological actions such as angiogenesis, increased vascular permeability and proliferation of vascular endothelial cells. Pharmaceuticals targeting VEGF have been already used widely in clinical use for the treatment of ocular diseases such as age-related macular degeneration (AMD), branch retinal vein occlusion (BRVO), central retinal vein occlusion (CRVO), diabetic maculopathy, diabetic retinopathy and neovascular glaucoma.

(2S,4S)-6-fluoro-2',5'-dioxospiro[chroman-4,4'-imidazolidine]-2-carboxamide (fidarestat) is an aldose reductase (AR) inhibitor. With respect to the compound, applications in diabetic complications (Patent Document 1), circulatory disorders (Patent Document 2), disorders associated with aging as a Maillard reaction inhibitor (Patent Document 3), simple diabetic retinopathy (Patent Document 4), diabetic keratopathy (Patent Document 5), diabetic maculopathy (Patent Document 6), severe diabetic retinopathy (Patent Document 7), cardiac functional disorders or myocardial disorders caused by ischemia or ischemic-reperfusion (Patent Document 8), acute renal failure (Patent Document 9), cerebral ischemia or cerebral ischemic-reperfusion injury in cerebral apoplexy (Patent Document 10) and an agent for protecting retinal neuron or optic nerve (Patent Document 11) are described in the respective Documents. And, it is described that the compound suppresses the retinal oxidative stress and the overexpression of VEGF in a streptozotocin (STZ)-diabetic rat (Non-patent Document 1).

On the other hand, it is known that anti-VEGF agents are used in combination with other pharmaceuticals (Patent Documents 12 and 13). For example, combination use with tumor necrosis factor alpha (TNFα) (Patent Documents 14 and 15), combination use with a receptor-type tyrosine kinase inhibitor (Patent Document 16), combination use with an antihypertensive drug (Patent Document 17), and combination use with a photosensitive drug used in a photodynamic therapy (PDT) (Patent Document 18) have been described. However, combination use of an anti-VEGF agent with an aldose reductase inhibitor has not been reported yet.

### Citation List

### Patent Literature

Patent Document 1: Japanese Patent Application Laid-Open Publication No. 61-200991
Patent Document 2: Japanese Patent Application Laid-Open Publication No. 4-173791
Patent Document 3: Japanese Patent Application Laid-Open Publication No. 6-135968
Patent Document 4: Japanese Patent Application Laid-Open Publication No. 7-242547
Patent Document 5: Japanese Patent Application Laid-Open Publication No. 8-231549
Patent Document 6: International Publication No. WO 2005/072066
Patent Document 7: International Publication No. WO 2005/079792
Patent Document 8: International Publication No. WO 2006/090699
Patent Document 9: International Publication No. WO 2007/069727
Patent Document 10: International Publication No. WO 2007/097301
Patent Document 11: International Publication No. WO 2008/093691
Patent Document 12: Japanese Patent Application National Publication (Laid-Open) No. 2007-505939
Patent Document 13: Japanese Patent Application National Publication (Laid-Open) No. 2007-505938
Patent Document 14: Japanese Patent Application Laid-Open Publication No. 2009-256373
Patent Document 15: Japanese Patent Application National Publication (Laid-Open) No. 2004-529149
Patent Document 16: Japanese Patent Application Laid-Open Publication No. 2009-102359
Patent Document 17: Japanese Patent Application National Publication (Laid-Open) No. 2008-537538
Patent Document 18: International Publication No. WO 2001/74389

### Non Patent Literature

Non-patent Document 1: Diabetes, Vol. 52, 864, 2003

### Summary of Invention

### Technical Problem

As mentioned above, although intravitreal injections of anti-VEGF agents are therapeutic agents that are widely used in clinical use, when the dose is increased in a patient having low satisfaction with treatment, they tend to cause side effects such as stroke. Furthermore, when the frequency of dosing increases, the emotional and physical burdens of patients and medical clinics increase, which tend to lead to the interruption of the treatment. For example, it is reported that ranibizumab that is a typical anti-VEGF agent has a short half-life and has a disappearing half-life of 9 days when it is injected in the vitreous body of a human (the package insert of ranibizumab). This leads to the increase in the frequency of administration of ranibizumab, which is heavy emotional, physical and medical economic burdens for patients and medical clinics. Therefore, a strategy for increasing the clinical effect of an anti-VEGF agent, and a strategy for decreasing the frequency of administration and dose of an anti-VEGF agent, thereof are demanded. Therefore, the present invention aims at decreasing the dose, decreasing the frequency of administration, and improving the efficiency, of an anti-VEGF agent.

### Solution to Problem

In view of the above-mentioned problem, the present inventors have done intensive studies and found that these objects can be achieved by combining an anti-VEGF agent and a hydantoin derivative represented by the following general formula (I) that is known as an aldose reductase inhibitor, specifically fidarestat, and completed the invention. Namely, the major constitutions of the present invention are as follows.
(1) A hydantoin derivative represented by a general formula (I) for use in preventing or treating a disorder accompanied by ocular angiogenesis and/or increased ocular vascular permeability in combination with an anti-VEGF agent: 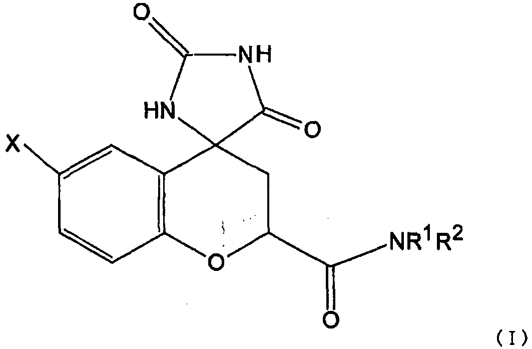 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof, wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.
(2) An anti-VEGF agent for use in preventing or treating a disorder accompanied by ocular angiogenesis and/or increased ocular vascular permeability in combination with a hydantoin derivative represented by a general formula (I): 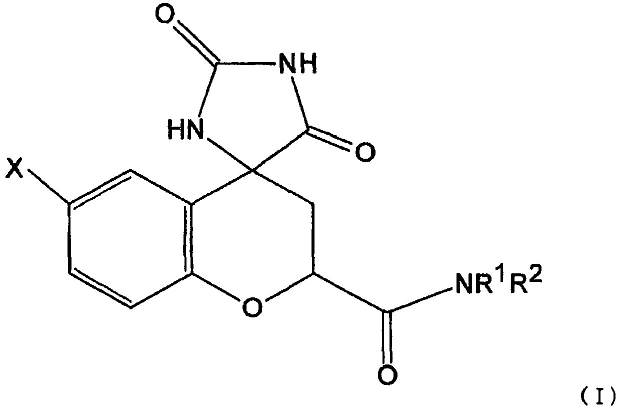 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof, wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.
(3) The compound for use according to (1) or (2) wherein the hydantoin derivative represented by the general formula (I) is (2S,4S)-6-fluoro-2',5'-dioxospiro[chroman-4,4'-imidazolidine]-2-carboxamide.
(4) The compound for use according to any one of (1) to (3) wherein the anti-VEGF agent is at least one kind selected from the group consisting of an anti-VEGF antibody, a VEGF Ligand inhibitor, a VEGF receptor antagonist and a oligonucleotide drug related to VEGF.
(5) The compound for use according to any one of (1) to (4) wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.
(6) A product comprising a hydantoin derivative represented by a general formula (I) and an anti-VEGF agent for simultaneous, separate or sequential use in the preventing or treating a disorder accompanied by ocular angiogenesis and/or increased ocular vascular permeability: 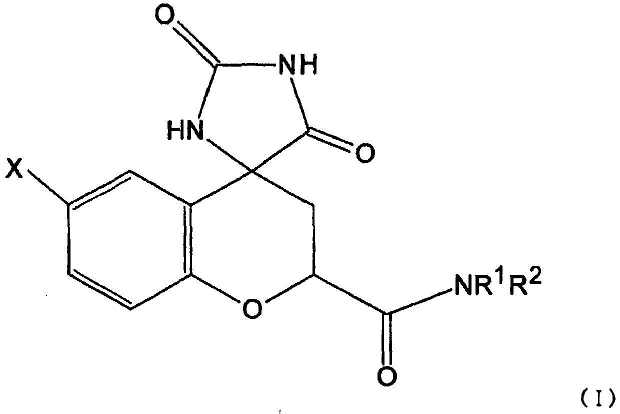 In the formula, X represents a halogen atom or a hydrogen atom, and R¹ and R² simultaneously or individually represent a hydrogen atom or a optionally substituted C₁₋₆ alkyl group, or a pharmacologically acceptable salt thereof, wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.
(7) The product for use according to (6) wherein the hydantoin derivative represented by the general formula (I) is (2S,4S)-6-fluoro-2',5'-dioxospiro[chroman-4,4'-imidazolidine]-2-carboxamide.
(8) The product for use according to (6) or (7) wherein the anti-VEGF agent is at least one kind selected from the group consisting of an anti-VEGF antibody, a VEGF Ligand inhibitor, a VEGF receptor antagonist and a oligonucleotide drug related to VEGF.
(9) The product for use according to any one of (6) to (8) wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.
(10) A pharmaceutical composition comprising a hydantoin derivative represented by a general formula (I) and an anti-VEGF agent for use in preventing or treating a disorder accompanied by ocular angionesis and/or increased ocular vascular permeability: 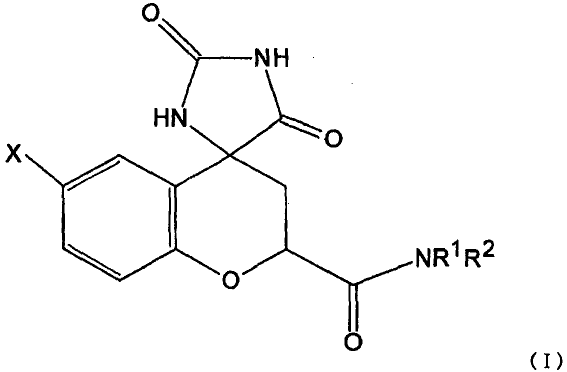 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof, wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.
(11) The pharmaceutical composition for use according to (10) wherein the hydantoin derivative represented by the general formula (I) is (2S,4S)-6-fluoro-2',5'-dioxospiro[chroman-4,4'-imidazolidine]-2-carboxamide.
(12) The pharmaceutical composition for use according to (10) or (11) wherein the anti-VEGF agent is at least one kind selected from the group consisting of an anti-VEGF antibody, a VEGF Ligand inhibitor, a VEGF receptor antagonist and a oligonucleotide drug related to VEGF.
(13) The pharmaceutical composition for use according to any one of (10) to (12) wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.
(14) A kit comprising: the compound for use according to (1) or (2), the product for use according to (6) or the pharmaceutical composition for use according to (10); and a written instruction describing a method for administering the pharmaceutical in combination with an anti-VEGF agent for preventing or treating a disorder accompanied by ocular angiogenesis and/or increased ocular vascular permeability.

### Advantageous Effects of Invention

The pharmaceutical of the present invention is effective for preventing or treating a disorder accompanied by excess ocular angiogenesis and/or increased ocular vascular permeability, and can decrease the dose, enhance the pharmacodynamic effect, extend the administration intervals and maintain the prolonged medicinal effect, of an anti-VEGF agent.

### Brief Description of Drawings

FIG. 1 is a graph showing the choroidal neovascularization (CNV) volume at 7 days after the irradiation of laser. The "*" and "#" in the drawing represent the presence of a significant difference at a risk rate of 5% and "**" represents the presence of a significant difference at a risk rate of 1%.
FIG 2 is a graph showing the rate of the number of grade 3 lesions in the fluorescein-fluorescent fundus angiography (FA) at 7 days after the irradiation of laser. The "***" in the drawing represents the presence of a significant difference at a risk rate of 0.1%.

### Description of Embodiments

Hereinafter the embodiments of the present invention will be explained in more detail. However, the present invention is not construed to be limited by the following embodiments.

The pharmaceutical according to the present invention includes a hydantoin derivative represented by the general formula (I) or a pharmacologically acceptable salt thereof as an active ingredient. This pharmaceutical is administered in combination with an anti-VEGF agent for preventing or treating a disorder accompanied by ocular angiogenesis and/or increased ocular vascular permeability. In other words, the pharmaceutical comprising a hydantoin derivative represented by the general formula (I) or a pharmacologically acceptable salt thereof as an active ingredient is used for an agent for enhancing the effect of the anti-VEGF agent for the prevention or treatment of a disorder accompanied by ocular angiogenesis and/or increased ocular vascular permeability.

The pharmaceutical according to the present invention is a pharmaceutical that is constituted by a combination of an anti-VEGF agent and a hydantoin derivative represented by the general formula (I) or a pharmacologically acceptable salt thereof. This pharmaceutical is also administered for preventing or treating a disorder accompanied by ocular angiogenesis and/or increased ocular vascular permeability.

The hydantoin derivative of the general formula (I) or a salt thereof is a compound having an aldose reductase inhibitory activity, and can be produced according to the synthesis method described in Japanese Patent Application Laid-Open Publication No. 63-057588.

X in the formula (I) represents a halogen atom or a hydrogen atom, preferably a halogen atom such as a fluorine atom. R¹ and R² in the formula (I) simultaneously or individually represent a hydrogen atom or an optionally substituted C₁₋₆ alkyl group, preferably a hydrogen atom or a C₁₋₃ alkyl group, and further preferably a hydrogen atom. Specifically, fidarestat that is a compound in which X is a fluorine atom and R¹ and R² are hydrogen atoms is excellent in medicinal effect and safeness.

VEGF are growth factor that acts on vascular endothelial cells. When VEGF bind to vascular endothelial cell growth factor receptor (VEGF receptor), vascular endothelial cells are activated, and the vascular endothelial cells migrate and proliferate to neovascularization. Furthermore, VEGF increase vascular permeability and cause hemorrhage, leakage of the components in blood and pooling of exudates.

Anti-VEGF agents are medicines that suppress the promoted angiogenesis by attenuating the activity of vascular endothelial growth factor (VEGF). Furthermore, in some cases, the anti-VEGF agents also show pharmacological actions such as suppression of vascular permeability and suppression of the proliferation of vascular endothelial cells. Examples of the anti-VEGF agents may include anti-VEGF antibodies, VEGF Ligand inhibitors, antagonists for VEGF receptors and oligonucleotide drugs related to VEGF. The antagonists for VEGF receptors antagonize VEGF to inhibit the binding of the VEGF to VEGF receptor. Anti-VEGF antibodies, VEGF Ligand inhibitors and some of oligonucleotide drugs related to VEGF inhibit the binding of VEGF to VEGF receptor by forming a conjugate with the VEGF. Other oligonucleotide drugs related to VEGF inhibit the synthesis of VEGF proteins. The anti-VEGF antibodies include anti-VEGF antibodies obtained by humanizing mouse anti-VEGF antibodies by gene recombination, fragments thereof, or those obtained by modifying a part of the amino acid sequences thereof. The VEGF Ligand inhibitors include human-type VEGF receptor fusion proteins.

The anti-VEGF antibodies, VEGF Ligand inhibitors, VEGF receptor antagonists and oligonucleotide drugs related to VEGF are common in that they all have an action of suppressing VEGF signals.

Specific examples of the VEGF Ligand inhibitors include aflibercept (VEGF-Trap) and VEGF-Trap EYE. Specific examples of the anti-VEGF antibodies include bevacizumab sodium, ranibizumab, sorafenib, and sunitinib. Specific examples of the oligonucleotide drugs related to VEGF include pegaptanib sodium that is an aptamer, and RTP801i-14 that is siRNA. In addition, bevacizumab sodium, ranibizumab and VEGF-Trap EYE and the like are characterized by that they inhibit the binding of VEGF isoforms to receptors by widely binding to all VEGF isoforms in a non-selective manner. On the other hand, pegaptanib sodium is characterized by that it selectively binds to VEGF 165 that is the most deeply involved in pathologic intraocular neovascular.

The medicinal effect that is exerted by administering the hydantoin derivative of the formula (I) or a salt thereof and the anti-VEGF agent in combination is within substantially the same of the medicinal effect that is exerted by administering the anti-VEGF agent alone. An embodiment of the pharmaceutical according to the present invention is a pharmaceutical that is effective for ocular disorders accompanied by excess angiogenesis, i.e., ocular neovascular diseases. Examples of the ocular neovascular diseases include ocular angiogenic disorders such as (wet) age-related macular degeneration (wet-AMD), branch retinal vein occlusion (BRVO), central retinal vein occlusion (CRVO), diabetic retinopathy, neovascular glaucoma, myopic choroidal neovascularization, retinitis pigmentosa, retinopathy of prematurity and photocoagulation-induced choroidal neovascularization. Another embodiment of the pharmaceutical according to the present invention is a pharmaceutical that is also effective for disorders accompanied by increased ocular vascular permeability. Examples of the disorders accompanied by increased ocular vascular permeability include wet age-related macular degeneration (wet-AMD), branch retinal vein occlusion (BRVO), central retinal vein occlusion (CRVO), diabetic maculopathy, retinitis pigmentosa, diabetic retinopathy and edema due to retinal photocoagulation (panretinal photocoagulation, grid photocoagulation, Grid Pattern photocoagulation). The pharmaceutical according to the present invention shows a synergic medicinal effect against these ocular angiogenic disorders and disorders accompanied by increased ocular vascular permeability for which administration of an anti-VEGF agent is considered to be effective.

Age-related macular degeneration is a disease caused by the degeneration in the macula associated with aging, and is classified into "wet" and "atrophic" according to the presence or absence of neovascularization that are generated from the choroid (choroidal neovascularization). The present invention is specifically effective for wet age-related macular degeneration.

Wet age-related macular degeneration is caused by generation of abnormal choroidal neovascularization (CNV) on sites centered around a macular area. These neovascular vessels may cause hemorrhage or exudation (increased vascular permeability), degenerate the tissue in the macula area relating to visual acuity, cause a malfunction and lead to scarring of the tissue. It is considered that VEGF is strongly involved in intraocular neovascular vessels.

Diabetic maculopathy is a generic term that includes impairment in the macular area caused by macular edema due to increased vascular permeability, and impairments in the macular area caused by occlusion of capillary vessels or impairment of pigment epithelial cells. The major lesion is macular edema. Since the amounts of VEGF are high in the eyes of a patient with macular edema and VEGF has an extremely strong action of elevating vascular permeability, it is considered that VEGF are strongly involved in macular edema in diabetic maculopathy.

The hydantoin derivative of the formula (I) and the anti-VEGF agent used as active ingredients of the pharmaceutical according to the present invention may be incorporated respectively in a plurality of separate formulations, or may be incorporated in the same formulation as one formulation. The "pharmaceutical composed of a combination (pharmaceutical comprising a combination)" is a pharmaceutical in which a plurality of drugs or the active ingredients thereof are administered in combination, in other words, administered by combination use. In the case when a plurality of active ingredients to be administered are incorporated in a plurality of separate formulations, these formulations are not necessarily administered at the same time to a patient. According to such administration by combination use, the effect of the combination use can be brought out sufficiently even when one of the pharmaceuticals (or the active ingredient thereof) does not maintain an effective concentration in blood or an effective concentration in tissue; therefore, it is not necessarily have to use an administration method in which the terms in which the respective pharmaceuticals maintain their effective concentrations in blood or effective concentrations in tissue are overlapped. In general, since the respective formulations are administered according to their original respective administration methods, the formulations may be administered by the same frequency or different frequencies.

Examples of the manners of administration of the above-mentioned pharmaceuticals may be those as listed below:
(1) administration of a single formulation comprising both the anti-VEGF agent and the hydantoin derivative represented by the general formula (I),
(2) simultaneous administration of two kinds of formulations obtained by separately formulating the anti-VEGF agent and the hydantoin derivative represented by the general formula (I) in the same administration route,
(3) staggered administration of two kinds of formulations obtained by separately formulating the anti-VEGF agent and the hydantoin derivative represented by the general formula (I) in the same administration route,
(4) simultaneous administration of two kinds of formulations obtained by separately formulating the anti-VEGF agent and the hydantoin derivative represented by the general formula (I) in different administration routes,
(5) staggered administration of two kinds of formulations obtained by separately formulating the anti-VEGF agent and the hydantoin derivative represented by the general formula (I) in different administration routes,
(6) administration of two kinds of formulations obtained by separately formulating the anti-VEGF agent and the hydantoin derivative represented by the general formula (I) in the same administration route at different administration intervals, and
(7) administration of two kinds of formulations obtained by separately formulating the anti-VEGF agent and the hydantoin derivative represented by the general formula (I) in different administration routes at different administration intervals. In addition, as the administration manner of (5), administration in the order that the anti-VEGF agent is first administered and then the hydantoin derivative represented by the general formula (I) is administered after a certain time period has passed, or administration by the inverse order can be exemplified. As the administration manner of (6) or (7), administration by the following administration intervals may be exemplified: administration by first administrating the anti-VEGF agent and then administrating the hydantoin derivative represented by the general formula (I) every day until the next administration of the anti-VEGF agent, or administration by first administrating the hydantoin derivative represented by the general formula (I) every day, then administrating the anti-VEGF agent, and subsequently administrating the hydantoin derivative described above every day.

The pharmaceutical according to the present invention is, whether it is a pharmaceutical comprising a plurality of active ingredients in a plurality of separate formulations or a single formulation including the active ingredients in the same formulation, formulated into, for example, a tablet, a capsule agent, a powder, a granular agent, a liquid agent or a syrup agent by a general formulation technique. The formulated pharmaceutical may be administered orally, or parenterally as an injection or an eye drop, or the like.

In the case of a solid agent, excipients that are pharmacologically acceptable in formulation, for example, starch, lactose, purified white soft sugar, glucose, crystalline cellulose, carboxycellulose, carboxymethyl cellulose, carboxyethyl cellulose, calcium phosphate, magnesium stearate and gum arabic, and where necessary, a lubricant, a binder, a disintegrating agent, a coating agent and a colorant, and the like can be incorporated. In the case of a liquid agent, a stabilizer, a dissolution aid, a suspending agent, an emulsifying agent, a buffer agent and a preservative, and the like can be used.

The dose of the pharmaceutical according to the present invention can be suitably selected according to the general dose of the anti-VEGF agent or the hydantoin derivative represented by the general formula (I) depending on the subject to be administered, administration route, intended disease, dosage form and the like.

The hydantoin derivative represented by the general formula (I), specifically fidarestat, is daily administered at once or in several portions by generally from 0.1 mg/day to 450 mg/day, preferably from 1 mg/day to 300 mg/day per an adult patient, depending on the symptom, age, method for administration, dosage form and the like. The dose in the case of oral administration is generally from 0.1 mg/day to 450 mg/day, preferably from 1 mg/day to 400 mg/day, more preferably from 1 mg/day to 200 mg/day per an adult patient.

The dose of the anti-VEGF agent differs depending on the agent. The dose of bevacizumab sodium is from 0.1 mg/time to 2.5 mg/time per an adult patient by intravitreous administration. The dose of pegaptanib is generally from 0.1 mg/time to 3 mg/time, preferably 0.3 mg/time per an adult patient by intravitreous administration. The dose of ranibizumab is generally from 0.1 mg/time to 0.5 mg/time, preferably 0.5 mg/time per an adult patient by intravitreous administration. The dose of VEGF-TRAP EYE is generally from 0.1 mg/time to 4 mg/time per an adult patient by intravitreous administration. The anti-VEGF agent is essentially administered once a month to once two to three months in the case of intravitreous administration. Alternatively, the agent is administered as necessary in the case when the lesion deteriorates or relapses. By administering the anti-VEGF agent in combination with the hydantoin derivative of the general formula (I), the dose of the anti-VEGF agent can be decreased and the frequency of administration can be reduced as compared to the case when the anti-VEGF agent is administered alone.

The pharmaceutical of the present invention can also constitute a kit by the pharmaceutical comprising the hydantoin derivative of the general formula (I), and a written instruction that describes a method for administering the pharmaceutical in combination with the anti-VEGF agent for preventing or treating a disorder accompanied by ocular angiogenesis and/or increased ocular vascular permeability. The written instruction in the kit includes descriptive texts regarding the usage and dosage and the like in the case when the pharmaceutical comprising the hydantoin derivative of the general formula (I) is administered in combination with the anti-VEGF agent. The usage and dosage thereof are as mentioned above.

### Examples

The present invention will be explained in more detail by the following experimental examples. However, these do not limit the present invention, and may be modified to the extent that they do not deviate from the intent of the present invention.

### 1. Experiment 1: preventive effect relating to suppression of angiogenesis and increased vascular permeability

### - Effect of combination use of anti-mouse VEGF antibody and fidarestat

- Using a mouse model in which choroidal neovascularization (hereinafter referred to as "CNV" model) is experimentally induced by laser irradiation (CNV model), the effectiveness against the volume of CNV was evaluated. In the CNV model, macrophages migrate by the initiation of an inflammation by laser irradiation. Since VEGF are produced, neovascular vessels are generated, and increased vascular permeability is observed as the result thereof, the CNV model is one of models that produce VEGF. In other words, the CNV model is an experimental model, which shows effectiveness against disorders accompanied by ocular angiogenesis and/or increased ocular vascular permeability through VEGF.

### Method

The both eyes of a male C57BL/6J mice having a body weight of about 20 g (6-week old) were irradiated with laser (irradiated by 4 to 6 spots per one eye) to induce CNV experimentally. As mentioned below, an anti-VEGF antibody and/or fidarestat were administered as a drug. The dose of fidarestat was 32 mg/kg/day, and fidarestat shows the maximum medicinal effect at this dose. As the anti-VEGF antibody, an anti-mouse VEGF antibody purchased from R&D Systems was used. The number of examples was three or four per a group, and the following four groups were set.
(1) Untreated group
(2) Fidarestat-administered group: free ingestion of a powder feedstuff comprising 32 mg/kg/day of fidarestat
(3) Vascular endothelial growth factor (VEGF)-neutralizing antibody-administered group: administration of 1 ng of the anti-VEGF antibody in the vitreous body
(4) Combination use group: ingestion of fidarestat in the same manner as that in "(2) Fidarestat-administered group" + administration of 1 ng of the anti-VEGF antibody into the vitreous body

Fidarestat was administered from 2 days before the laser irradiation to 7 days after the irradiation, and the anti-VEGF antibody was administered in the vitreous body immediately after the laser irradiation. After completion of the laser irradiation, the mice were bred by general breeding for 7 days, and thereafter the eye balls were excised and the volume of CNV appeared in the choroid was measured. The method for the induction and evaluation of CNV are as follows.

### [Induction of experimental CNV by laser irradiation]

The mice were anesthetized by administering 0.30 mL of tribromoethanol to the abdominal cavity of the mice, and a mydriatics and a local anesthesia were placed a drop of the eyes. Thereafter the both eyes of the mice were irradiated with laser (wavelength: 532 nm, output: 200 mW, irradiation time: 100 ms, spot size: 100 µm) by 4 to 6 spots per one eye using a laser photocoagulation device (Lumenis). CNV appeared by the laser irradiation, and thereafter the volume of CNV was increased.

### [Evaluation of CNV volume]

At 7 days after the laser irradiation, the mice were euthanized under tribromoethanol anesthesia, and the left and right eye balls of the mice were excised. Using the excised eye balls, flat mounts were prepared according to a conventional method. CNV was stained by using FITC-Griffonia simplicifolia isolectin-B4. The volume of CNV was measured by importing an image in the vicinity of CNV by a confocal laser microscope (Zeiss LSM5 Pascal) and using image analysis software (NIH Image J). The volume was represented by µm³.

### Result

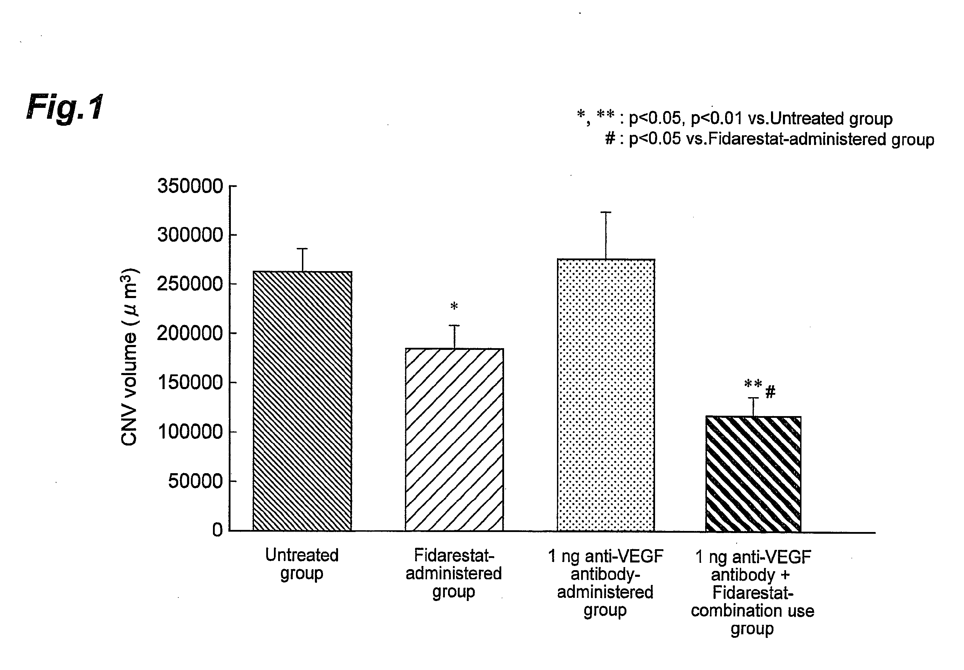

The choroidal neovascularization (CNV) volume at 7 days after the laser irradiation is shown in FIG. 1. A significant effect of suppressing CNV was not observed in the 1 ng anti-VEGF antibody-administered group as compared to the untreated group. A significant effect of suppressing CNV was observed (p<0.05) in the fidarestat-administered group as compared to the untreated group. On the other hand, a striking effect of suppressing CNV was observed (p<0.01) in the group of combination use of fidarestat and 1 ng of the anti-VEGF antibody as compared to the untreated group.

When fidarestat was used in combination with a dose of 1 ng that was decreased to one-tenth of 10 ng that is the dose at which the anti-VEGF antibody shows the maximum medicinal effect, the effect thereof was enhanced significantly, and thus a striking effect of suppressing CNV was observed even at a low dose.

### Consideration

The result at this time shows that, when fidarestat is used in combination with an anti-VEGF agent at a low dose which does not show effectiveness against CNV by itself, an extremely striking effect of combination use can be observed as compared to the cases when each of them is administered alone. Since the dose at which the anti-VEGF antibody shows the maximum medicinal effect is 10 ng, it becomes possible to decrease the dosage of the anti-VEGF agent to one-tenth by this combination use, and thus it is expected that this decrease lead to the decrease in the side effects of the anti-VEGF agent.

The present result also shows that the effect is enhanced even fidarestat is used in combination in the state that the clinical effect has been attenuated or diminished due to the decrease in the concentration in blood or concentration in tissue of the anti-VEGF antibody. This result means that the effect is not attenuated by using fidarestat in combination even the concentration of the anti-VEGF agent in the vitreous body is decreased to one-tenth or less. According to the enhancement of the effect by such combination use, the maintenance of the prolonged medical effect of the anti-VEGF agent, and the time interval until the attenuation of the effect or re-administration due to recrudescence are extended, which leads to the decrease in the frequency of injection of the anti-VEGF agent to the vitreous body. Namely, the result in the combination use group at this time further enhances the effectiveness in a patient for which a therapeutic effect has been clinically observed, and consequently leads to the extension of the time interval until the re-administration of the anti-VEGF agent, i.e., maintenance of the prolonged medical effect. Furthermore, an excellent effect of suppressing angiogenesis can be expected by combination use even in a patient for which the effect of a treatment with an existing anti-VEGF agent is insufficient. Alternatively, it can be expected that a sufficient therapeutic effect is achieved even the dose of the anti-VEGF agent is decreased.

Meanwhile, the anti-mouse VEGF antibody used in the present experiment is common with clinically-used anti-VEGF antibodies in that it inhibits all VEGF isoforms. Therefore, the effect of the anti-mouse VEGF antibody can be read as the effect of a human-type anti-VEGF antibody when used in a human.

### 2. Experiment 2: therapeutic effect against increased vascular permeability

### - Effect of combination use of anti-mouse VEGF antibody and fidarestat

### Method

An anti-VEGF antibody and/or fidarestat were administered to a mouse that had been treated to induce CNV in a similar manner to that in Experiment 1. 10 ng of the anti-VEGF antibody is the dose at which the antibody shows the maximum medicinal effect. The number of examples was three or four per a group, and the following five groups were set.
(1) Untreated group
(2) Fidarestat-administered group: forced oral administration of 16 mg/kg of fidarestat twice a day (32 mg/kg/day))
(3) 2 ng anti-VEGF antibody-administered group: administration of 2 ng of the anti-VEGF antibody to the vitreous body
(4) 10 ng anti-VEGF antibody-administered group: administration of 10 ng of the anti-VEGF antibody to the vitreous body
(5) Combination use group: administration of 2 ng of the anti-VEGF antibody to the vitreous body + administration of fidarestat (forced oral administration of 16 mg/kg twice a day (32 mg/kg/day))

Fidarestat was administered from the day of laser irradiation (20 to 30 minutes after the irradiation) to 7 days after the irradiation, and the anti-VEGF antibody was administered in the vitreous body immediately after the laser irradiation. After completion of the laser irradiation, the mice were bred by general breeding for 7 days, and fluorescein fundus angiography (FA) was photographed. The method for fluorescein-fluorescent fundus angiography is as follows.

### [Fluorescein-fluorescent fundus angiography]

At 7 days after the laser irradiation, 0.1 mL of 1% fluorescein was administered intraperitoneally under tribromoethanol anesthesia. The both eyes were photographed by an eye-fundus camera, and the obtained eye-ground picture was evaluated in a blinded manner according to the following criteria.
grade 0: nonleaky, no leakage from blood-vessel
grade 1: questionable leakage, weak leakage was observed
grade 2: leaky, leakage was observed
grade 3: pathologically significant leakage, clear leakage was observed on wide area

The ratio of the number of the FA grade 3 lesions (%) in each eye was calculated, and the average value of the calculated rates in each group was obtained. When an eye-fundus image was photographed after administering a fluoro chrome (fluorescein), the vascular network can be observed clearly. In a normal case, leakage of the blood components from the blood-vessel is not observed, whereas when the blood-vessel is abnormal, leakage out of the blood-vessel is observed.

### Result

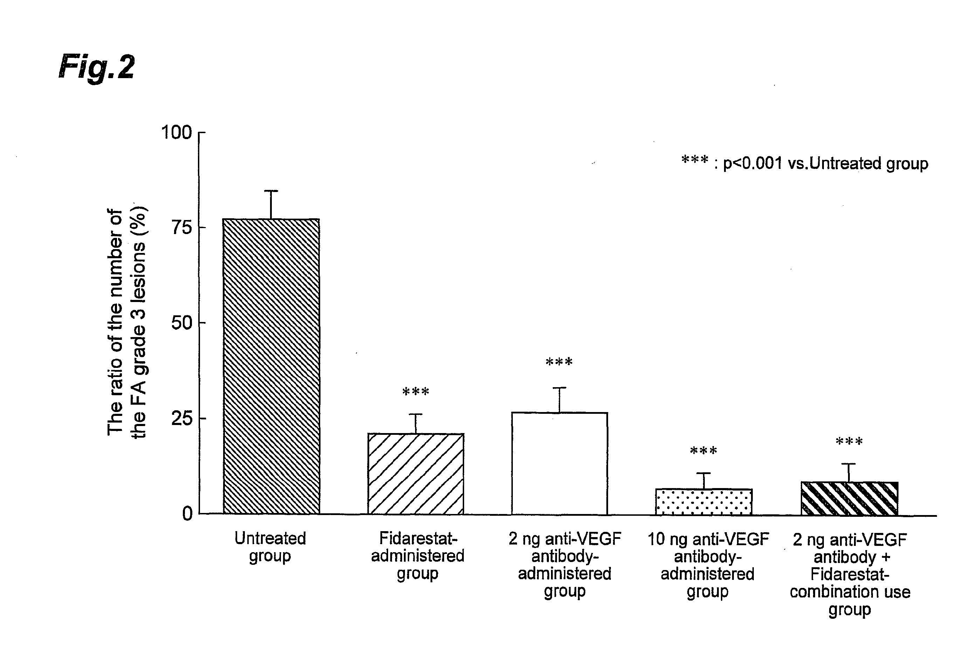

The ratio of the number of the FA grade 3 lesions (%) in the fluorescein-fluorescent fundus angiography is shown in FIG. 2. In the anti-VEGF antibody-administered group, significant decrease in the rate of the number of the lesions (p<0.001) was observed in a dose-dependent manner. In the 10 ng anti-VEGF antibody-administered group, the rate of the lesion number was 6%. Therefore, it is understood that the vascular permeability in the present model is a model to which VEGF contribute. On the other hand, in the 2 ng anti-VEGF antibody-administered group, the rate thereof was 26%. Although significant decrease was also observed in the fidarestat-administered group, it showed approximately the same rate as that in the 2 ng anti-VEGF antibody-administered group. In the group of combination use of 2 ng anti-VEGF antibody + fidarestat, the effect thereof was enhanced by three times or more, and a similar suppression effect to that in the 10 ng anti-VEGF antibody-administered group was observed.

### Consideration

It was shown that, despite the fact that the ratio of the number of the FA grade 3 lesions in the fidarestat-administered group was approximately the same as that in the 2 ng anti-VEGF antibody-administered group, when they are used in combination, the ratio was decreased to one-third or less of that in the 2 ng anti-VEGF antibody-administered group. The effect of decreasing the ratio of the number of the FA grade 3 lesions by this combination use was an almost complete effect of suppressing vascular permeability that is comparable to the maximum effect of the anti-VEGF antibody. It is generally expected that when two kinds of anti-VEGF agents are used in combination by doses at which similar decreasing effects are shown, the ratio of the number of the FA grade 3 lesions is decreased to only half as compared to the case when one kind of anti-VEGF agent is administered, whereas in the present result, the ratio was decreased to a ratio that goes beyond the expectation. Furthermore, it is surprising that the result showed that a similar effect to the maximum medicinal effect of the anti-VEGF antibody can be obtained by combination use with fidarestat even the dose of the anti-VEGF antibody is decreased to one-fifth.

The present result shows that an excellent suppression effect against not only angiogenesis but also extravasation can be obtained by using the anti-VEGF agent and fidarestat in combination. Extravasation, i.e., increased vascular permeability means appearance of edema, and suppression of this strongly suggests the effectiveness on edema lesions. Specifically, since the elimination half-life of anti-VEGF agent in the vitreous body is short, it has a problem in the maintenance of a prolonged clinical effect and needs to be injected frequently in the vitreous body. It was suggested that the effect on edema lesions based on increased vascular permeability is sustained by using fidarestat in combination, which consequently enables extension of the time interval until the re-administration of the anti-VEGF agent by vitreous injection, i.e., enables decreasing of the frequency of the re-administration.

### Summary

The effect of the anti-VEGF agent becomes the maximum at the time when abnormal VEGF production has been suppressed completely or when VEGF signals have been suppressed completely. Namely, it is considered that the effect becomes the maximum if one of the production system or signal system is suppressed completely, and further enhancement of the effect cannot be expected even another system is further suppressed at this time.

10 ng of the anti-VEGF antibody is considered to be a dose at which the maximum medicinal effect is shown when VEGF signals are suppressed. Furthermore, 32 mg/kg of fidarestat is also considered to be a dose at which the maximum medicinal effect is shown when abnormal VEGF production is suppressed. Therefore, persons skilled in the art would expect that, even the anti-VEGF antibody is added to, for example, 32 mg/kg of fidarestat, the effect thereof is not enhanced.

However, contrary to such expectation, a further strong effect was observed by combination use of fidarestat and the anti-VEGF antibody in Experiments 1 and 2, as compared to the group in which fidarestat was administered by a dose at which fidarestat alone shows the maximum medicinal effect. These results show that an effect that cannot be achieved by only increasing the dose of a single drug can be achieved by combination use.

The fact that such effect is exerted cannot be explained by only the effect of suppressing VEGF. It is considered that some kind of action for suppressing the mechanism of angiogenesis and/or increased vascular permeability, which is different from VEGF, operates. Such action that is equal to or more than the maximum medicinal effect against VEGF goes beyond the expectation of persons skilled in the art in any of non-diabetic diseases and diabetes mellitus.

## Claims

1. A hydantoin derivative represented by a general formula (I) for use in preventing or treating a disorder accompanied by ocular angiogenesis and/or increased ocular vascular permeability in combination with an anti-VEGF agent: 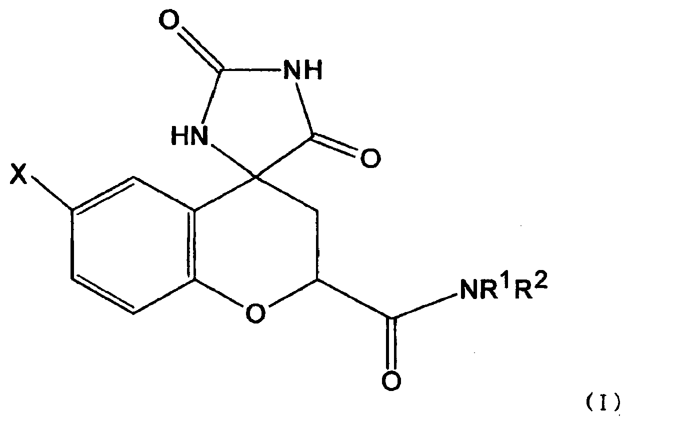 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof,
wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.

2. An anti-VEGF agent for use in preventing or treating a disorder accompanied by ocular angiogenesis and/or increased ocular vascular permeability in combination with a hydantoin derivative represented by a general formula (I): 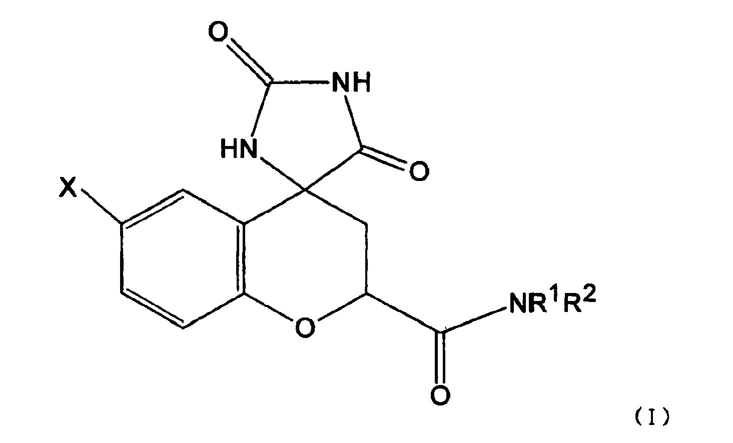 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof,
wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.

3. The compound for use according to claim 1 or 2 wherein the hydantoin derivative represented by the general formula (I) is (2S,4S)-6-fluoro-2',5'-dioxospiro[chroman-4,4'-imidazolidine]-2-carbox amide.

4. The compound for use according to any one of claims 1 to 3 wherein the anti-VEGF agent is at least one kind selected from the group consisting of an anti-VEGF antibody, a VEGF Ligand inhibitor, a VEGF receptor antagonist and a oligonucleotide drug related to VEGF.

5. The compound for use according to any one of claims 1 to 4 wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.

6. A product comprising a hydantoin derivative represented by a general formula (I) and an anti-VEGF agent for simultaneous, separate or sequential use in the preventing or treating a disorder accompanied by ocular angiogenesis and/or increased ocular vascular permeability: 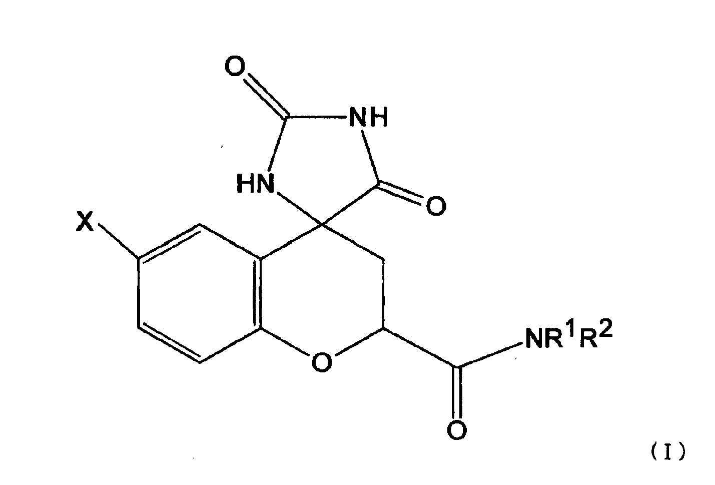 In the formula, X represents a halogen atom or a hydrogen atom, and R¹ and R² simultaneously or individually represent a hydrogen atom or a optionally substituted C₁₋₆ alkyl group, or a pharmacologically acceptable salt thereof,
wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.

7. The product for use according to claim 6 wherein the hydantoin derivative represented by the general formula (I) is (2S,4S)-6-fluoro-2',5'-dioxospiro[chroman-4,4'-imidazolidine]-2-carbox amide.

8. The product for use according to claim 6 or 7 wherein the anti-VEGF agent is at least one kind selected from the group consisting of an anti-VEGF antibody, a VEGF Ligand inhibitor, a VEGF receptor antagonist and a oligonucleotide drug related to VEGF.

9. The product for use according to any one of claims 6 to 8 wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.

10. A pharmaceutical composition comprising a hydantoin derivative represented by a general formula (I) and an anti-VEGF agent for use in preventing or treating a disorder accompanied by ocular angionesis and/or increased ocular vascular permeability: 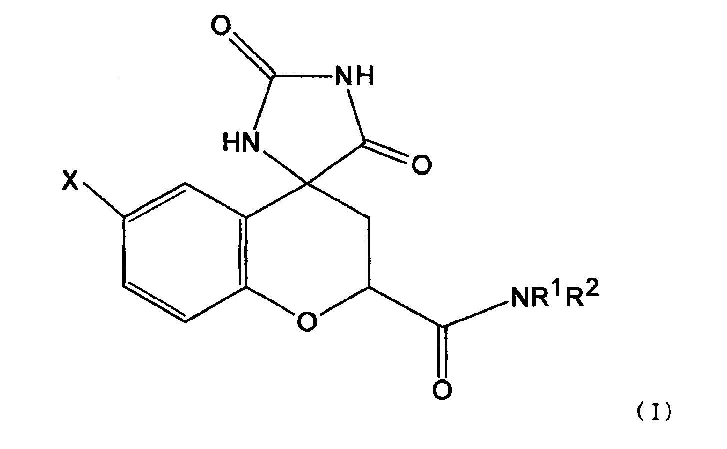 in the formula, X represents a halogen atom or a hydrogen atom, and R¹ and R² simultaneously or individually represent a hydrogen atom or an optionally substituted C₁₋₆ alkyl group, or a pharmacologically acceptable salt thereof,
wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma, myopic choroidal neovascularization, retinitis pigmentosa, retinopathy of prematurity, photocoagulation-induces choroidal neovascularization, and edema due to retinal photocoagulation.

11. The pharmaceutical composition for use according to claim 10 wherein the hydantoin derivative represented by the general formula (I) is (2S,4S)-6-fluoro-2',5'-dioxospiro[chroman-4,4'-imidazolidine]-2-carbo xamide.

12. The pharmaceutical composition for use according to claim 10 or 11 wherein the anti-VEGF agent is at least one kind selected from the group consisting of an anti-VEGF antibody, a VEGF Ligand inhibitor, a VEGF receptor antagonist and a oligonucleotide drug related to VEGF.

13. The pharmaceutical composition for use according to any one of claims 10 to 12 wherein the disorder accompanied by ocular angiogenesis and/or increased ocular vascular permeability is selected from the group consisting of age-related macular degeneration, branch retinal vein occlusion, central retinal vein occlusion, diabetic maculopathy, diabetic retinopathy, neovascular glaucoma.

14. A kit comprising:
the compound for use according to claim 1 or 2, the product for use according to claim 6 or the pharmaceutical composition for use according to claim 10; and
a written instruction describing a method for administering the pharmaceutical in combination with an anti-VEGF agent for preventing or treating a disorder accompanied by ocular angiogenesis and/or increased ocular vascular permeability.

## Patentansprüche

1. Hydantonin-Derivat gemäß der allgemeinen Formel (I) für die Verwendung in der Prävention oder Behandlung von einer Störung begleitet von einer okularen Angiogenesis und/oder einer erhöhten okularen Gefäßpermeabilität in Kombination mit einem Anti-VEGF-Wi rkstoff: 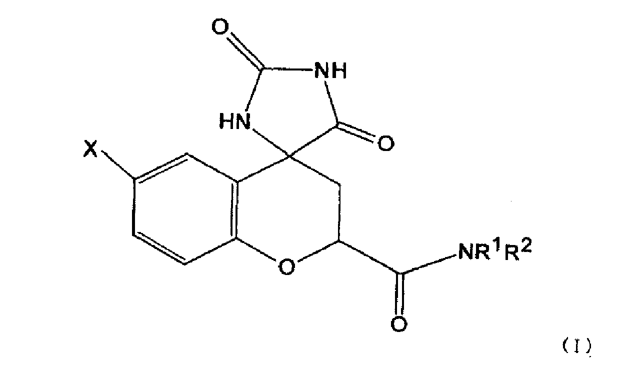 in der Formel ist X ein Halogenatom oder ein Wasserstoffatom und R1 und R2 gleichzeitig oder individuell ein Wasserstoffatom oder eine ggfs. substituierte C₁₋₆-Alkylgruppe oder ein pharmakologisch annehmbares Salz davon,
wobei die Störung begleitet von einer okularen Angiogenesis und/oder einer erhöhten okularen Gefäßpermeabilität ausgewählt ist aus der Gruppe bestehend aus altersbedingter makularer Degeneration, retinaler Astvenenverschluss, retinalem Zentralvenenverschluss, diabetische Makulopathie, diabetische Retinopathie, myopische choroidale Neovaskularisation, Retinitis Pigmentosa, Frühgeborenen-Retinopathie, durch Photokoagulation induzierte choroidale Neovaskularisation und Ödeme aufgrund von retinaler Photokoagulation.

2. Anti-VEGF-Wirkstoff für die Verwendung in der Prävention oder Behandlung einer Störung begleitet von okularer Angiogenesis und/oder erhöhter okularer Gefäßpermeabilität in Kombination mit einem Hydantonin-Derivat gemäß der allgemeinen Formel (I): 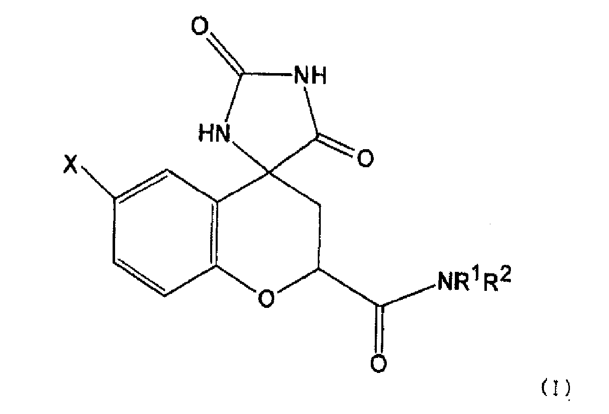 in der Formel ist X ein Halogenatom oder ein Wasserstoffatom und R1 und R2 gleichzeitig oder individuell ein Wasserstoffatom oder eine ggfs. substituierte C₁₋₆-Alkylgruppe oder ein pharmakologisch annehmbares Salz davon,
wobei die Störung begleitet von einer okularen Angiogenesis und/oder einer erhöhten okularen Gefäßpermeabilität ausgewählt ist aus der Gruppe bestehend aus altersbedingter makularer Degeneration, retinalem Astvenenverschluss, retinalem Zentralvenenverschluss, diabetische Makulopathie, diabetische Retinopathie, myopische choroidale Neovaskularisation, Retinitis Pigmentosa, Frühgeborenen-Retinopathie, durch Photokoagulation induzierte choroidale Neovaskularisation und Ödeme aufgrund von retinaler Photokoagulation.

3. Verbindung für die Verwendung gemäß Anspruch 1 oder 2, wobei das Hydantonin-Derivat gemäß der allgemeinen Formel (I) (2S,4S)-6-Fluor-2',5'-dioxospiro[chroman-4,4'-imidazolidin]-2-carboxamid ist.

4. Verbindung zur Verwendung nach einem der Ansprüche 1 bis 3, wobei der Anti-VEGF-Wirkstoff mindestens eine Sorte ausgewählt aus der Gruppe bestehend aus einem Anti-VEGF-Antikörper, einem VEGF-Ligand-Inhibitor, einem VEGF-Rezeptor-Antagonist und einem Oligonukleotid-Arzneimittel in Verbindung mit VEGF ist.

5. Verbindung zur Verwendung nach einem der Ansprüche 1 bis 4, wobei die Störung begleitet von okularer Angiogenesis und/oder erhöhter okularer Gefäßpermeabilität ausgewählt ist aus der Gruppe bestehend aus altersbedingter makularer Degeneration, retinalem Astvenenverschluss, retinalem Zentralvenenverschluss, diabetischer Makulopathie, diabetische Retinopathie, neovaskuläres Glaukom.

6. Ein Produkt enthaltend ein Hydantonin-Derivat gemäß der allgemeinen Formel (I) und einem Anti-VEGF-Wirkstoff für die gleichzeitige, separate oder sequentielle Verwendung in der Prävention und Behandlung einer Störung begleitet durch okulare Angiogenesis und/oder erhöhter okularer Gefäßpermeabilität: 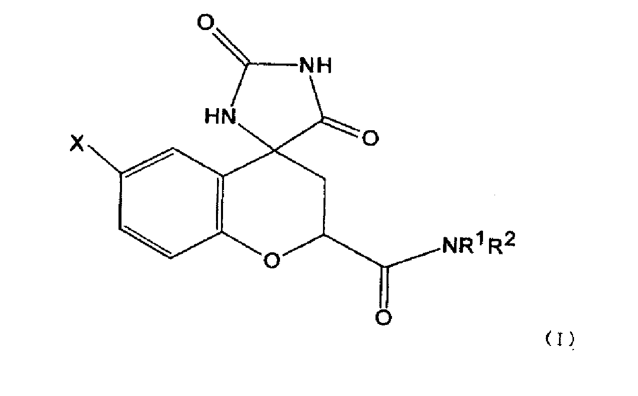 in der Formel ist X ein Halogenatom oder ein Wasserstoffatom und R1 und R2 gleichzeitig oder individuell ein Wasserstoffatom oder eine ggfs. substituierte C₁₋₆-Alkylgruppe oder ein pharmakologisch annehmbares Salz davon,
wobei die Störung begleitet von einer okularen Angiogenesis und/oder einer erhöhten okularen Gefäßpermeabilität ausgewählt ist aus der Gruppe bestehend aus altersbedingter makularer Degeneration, retinalem Astvenenverschluss, retinalem Zentralvenenverschluss, diabetische Makulopathie, diabetische Retinopathie, myopische choroidale Neovaskularisation, Retinitis Pigmentosa, Frühgeborenen-Retinopathie, durch Photokoagulation induzierte choroidale Neovaskularisation und Ödeme aufgrund von retinaler Photokoagulation.

7. Produkt zur Verwendung gemäß Anspruch 6, wobei das Hydantonin-Derivat gemäß der allgemeinen Formel (I) (2S,4S)-6-Fluor-2',5'-dioxospiro[chroman-4,4'-imidazolidin]-2-carboxamid ist.

8. Produkt zur Verwendung gemäß Anspruch 6 oder 7, wobei der Anti-VEGF-Wirkstoff mindestens eine Sorte ausgewählt aus der Gruppe bestehend aus einem Anti-VEGF-Antikörper, einem VEGF-Ligand-Inhibitor, einem VEGF-Rezeptor-Antagonist und einem Oligonukleotid-Arzneimittel in Verbindung mit VEGF ist.

9. Produkt zur Verwendung nach einem der Ansprüche 6 bis 8, wobei die Störung begleitet von okularer Angiogenesis und/oder erhöhter okularer Gefäßpermeabilität ausgewählt ist aus der Gruppe bestehend aus altersbedingter makularer Degeneration, retinalem Astvenenverschluss, retinalem Zentralvenenverschluss, diabetischer Makulopathie, diabetische Retinopathie, neovaskuläres Glaukom.

10. Pharmazeutische Zusammensetzung enthaltend ein Hydantonin-Derivat gemäß einer allgemeinen Formel (I) und einem Anti-VEGF-Wirkstoff für die Verwendung in der Prävention oder Behandlung einer Störung begleitet durch okularer Angiogenesis und/oder erhöhte okulare Gefäßpermeabilität: 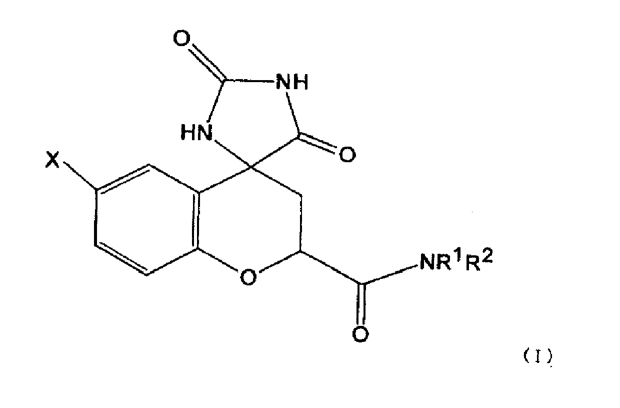 in der Formel ist X ein Halogenatom oder ein Wasserstoffatom und R1 und R2 gleichzeitig oder individuell ein Wasserstoffatom oder eine ggfs. substituierte C₁₋₆-Alkylgruppe oder ein pharmakologisch annehmbares Salz davon,
wobei die Störung begleitet von einer okularen Angiogenesis und/oder einer erhöhten okularen Gefäßpermeabilität ausgewählt ist aus der Gruppe bestehend aus altersbedingter makularer Degeneration, retinalem Astvenenverschluss, retinalem Zentralvenenverschluss, diabetische Makulopathie, diabetische Retinopathie, myopische choroidale Neovaskularisation, Retinitis Pigmentosa, Frühgeborenen-Retinopathie, durch Photokoagulation induzierte choroidale Neovaskularisation und Ödeme aufgrund von retinaler Photokoagulation.

11. Pharmazeutische Zusammensetzung zur Verwendung gemäß Anspruch 10, wobei das Hydantonin-Derivat gemäß der allgemeinen Formel (I) (2S,4S)-6-Fluor-2',5'-dioxospiro[chroman-4,4'-imidazolidin]-2-carboxamid ist.

12. Pharmazeutische Zusammensetzung zur Verwendung gemäß Anspruch 10 oder 11, wobei der Anti-VEGF-Wirkstoff mindestens eine Sorte ausgewählt aus der Gruppe bestehend aus einem Anti-VEGF-Antikörper, einem VEGF-Ligand-Inhibitor, einem VEGF-Rezeptor-Antagonist und einem Oligonukleotid-Arzneimittel in Verbindung mit VEGF ist.

13. Pharmazeutische Zusammensetzung zur Verwendung gemäß einem der Ansprüche 10 bis 12, wobei die Störung begleitet von okularer Angiogenesis und/oder erhöhter okularer Gefäßpermeabilität ausgewählt ist aus der Gruppe bestehend aus altersbedingter makularer Degeneration, retinalem Astvenenverschluss, retinalem Zentralvenenverschluss, diabetischer Makulopathie, diabetische Retinopathie, neovaskuläres Glaukom.

14. Kit enthaltend: die Verbindung zur Verwendung gemäß Anspruch 1 oder 2, das Produkt zur Verwendung gemäß Anspruch 6 oder die pharmazeutische Zusammensetzung zur Verwendung gemäß Anspruch 10 und eine schriftliche Anleitung, die ein Verfahren zur Verabreichung des Arzneistoffs in Kombination mit einem Anti-VEGF-Wirkstoff für die Prävention und Behandlung einer Störung begleitet durch okulare Angiogenesis und/oder erhöhte okulare Gefäßpermeabilität beschreibt.

## Revendications

1. Dérivé de l'hydantoïne représenté par une formule générale (I) destiné à être utilisé dans la prévention ou le traitement d'un trouble accompagné par une angiogenèse oculaire et/ou une perméabilité vasculaire oculaire accrue en combinaison avec un agent anti-VEGF : 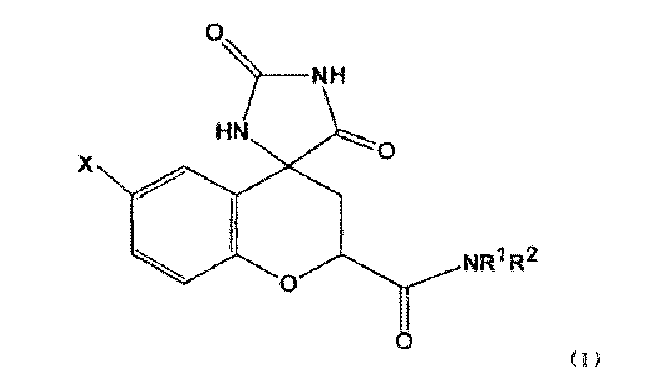 dans la formule, X représente un atome d'halogène ou un atome d'hydrogène, et R¹ et R² représentent simultanément ou individuellement un atome d'hydrogène ou un groupe C₁₋₆ alkyle éventuellement substitué, ou sel pharmacologiquement acceptable de celui-ci,
où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire, la néovascularisation choroïdienne myope, la rétinite pigmentaire, la rétinopathie de prématurité, la néovascularisation coroïdienne induite par photocoagulation et l'oedème dû à la photocoagulation rétinienne.

2. Agent anti-VEGF destiné à être utilisé dans la prévention ou le traitement d'un trouble accompagné par une angiogenèse oculaire et/ou une perméabilité vasculaire oculaire accrue en combinaison avec un dérivé de l'hydantoïne représenté par une formule générale (I) : 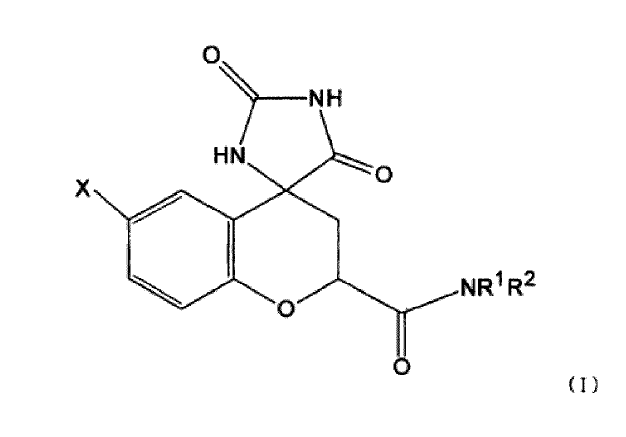 dans la formule, X représente un atome d'halogène ou un atome d'hydrogène, et R¹ et R² représentent simultanément ou individuellement un atome d'hydrogène ou un groupe C₁₋₆ alkyle éventuellement substitué, ou un sel pharmacologiquement acceptable de celui-ci,
où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire, la néovascularisation choroïdienne myope, la rétinite pigmentaire, la rétinopathie de prématurité, la néovascularisation coroïdienne induite par photocoagulation et l'oedème dû à la photocoagulation rétinienne.

3. Composé destiné à être utilisé selon la revendication 1 ou 2, où le dérivé de l'hydantoïne représenté par la formule générale (I) est le (2S,4S)-6-fluoro-2',5'-dioxospiro[chroman-4,4'-imidazolidine]-2-carboxamide.

4. Composé destiné à être utilisé selon l'une quelconque des revendications 1 à 3, où l'agent anti-VEGF est au moins un type choisi dans le groupe consistant en un anticorps anti-VEGF, un inhibiteur de ligand de VEGF, un antagoniste de récepteur de VEGF, et un médicament oligonucléotidique apparenté à VEGF.

5. Composé destiné à être utilisé selon l'une quelconque des revendications 1 à 4, où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire.

6. Produit comprenant un dérivé de l'hydantoïne représenté par une formule générale (I) et un agent anti-VEGF pour l'utilisation simultanée, séparée ou successive dans la prévention ou le traitement d'un trouble accompagné par une angiogenèse oculaire et/ou une perméabilité vasculaire oculaire accrue : 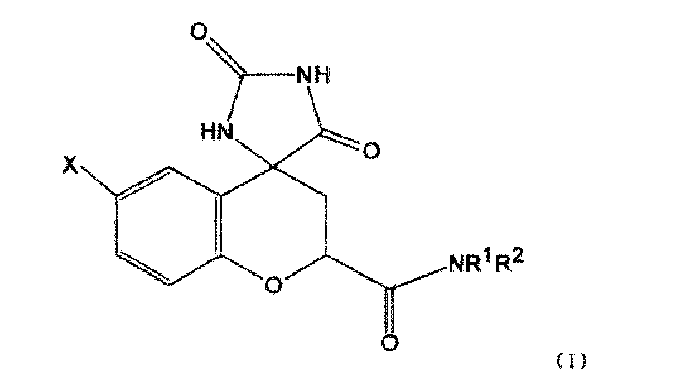 dans la formule, X représente un atome d'halogène ou un atome d'hydrogène, et R¹ et R² représentent simultanément ou individuellement un atome d'hydrogène ou un groupe C₁₋₆ alkyle éventuellement substitué, ou un sel pharmacologiquement acceptable de celui-ci,
où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire, la néovascularisation choroïdienne myope, la rétinite pigmentaire, la rétinopathie de prématurité, la néovascularisation coroïdienne induite par photocoagulation et l'oedème dû à la photocoagulation rétinienne.

7. Produit destiné à être utilisé selon la revendication 6, où le dérivé de l'hydantoïne représenté par la formule générale (I) est le (2S,4S)-6-fluoro-2',5'-dioxospiro[chroman-4,4'-imidazolidine]-2-carboxamide.

8. Produit destiné à être utilisé selon la revendication 6 ou 7, où l'agent anti-VEGF est au moins un type choisi dans le groupe consistant en un anticorps anti-VEGF, un inhibiteur de ligand de VEGF, un antagoniste de récepteur de VEGF et un médicament oligonucléotidique apparenté à VEGF.

9. Produit destiné à être utilisé selon l'une quelconque des revendications 6 à 8, où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire.

10. Composition pharmaceutique comprenant un dérivé de l'hydantoïne représenté par une formule générale (I) et un agent anti-VEGF destinée à être utilisée dans la prévention ou le traitement d'un trouble accompagné par une angiogenèse oculaire et/ou une perméabilité vasculaire oculaire accrue : 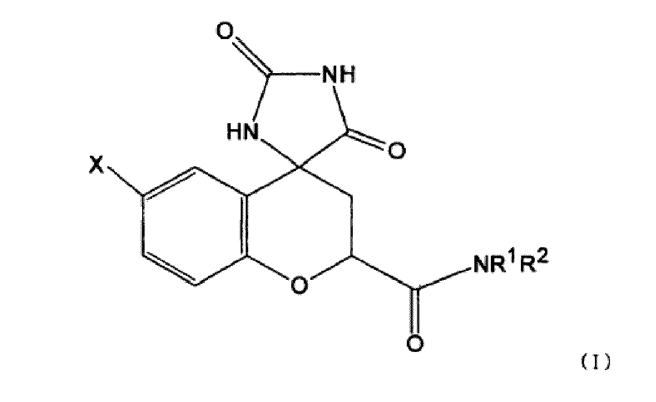 dans la formule, X représente un atome d'halogène ou un atome d'hydrogène, et R¹ et R² représentent simultanément ou individuellement un atome d'hydrogène ou un groupe C₁₋₆ alkyle éventuellement substitué, ou un sel pharmacologiquement acceptable de celui-ci,
où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire, la néovascularisation choroïdienne myope, la rétinite pigmentaire, la rétinopathie de prématurité, la néovascularisation coroïdienne induite par photocoagulation et l'oedème dû à la photocoagulation rétinienne.

11. Composition pharmaceutique destinée à être utilisée selon la revendication 10, où le dérivé de l'hydantoïne représenté par la formule générale (I) est le (2S,4S)-6-fluoro-2',5'-dioxospiro[chroman-4,4'-imidazolidine]-2-carboxamide.

12. Composition pharmaceutique destinée à être utilisée selon la revendication 10 ou 11, où l'agent anti-VEGF est au moins un type choisi dans le groupe consistant en un anticorps anti-VEGF, un inhibiteur de ligand de VEGF, un antagoniste de récepteur de VEGF, et un médicament oligonucléotidique apparenté à VEGF.

13. Composition pharmaceutique destinée à être utilisée selon l'une quelconque des revendications 10 à 12, où le trouble accompagné par une angiogenèse oculaire et/ou une perméabilité vasculaire oculaire accrue est choisi dans le groupe consistant en la dégénérescence maculaire liée à l'âge, l'occlusion de la veine rétinienne secondaire, l'occlusion de la veine rétinienne centrale, la maculopathie diabétique, la rétinopathie diabétique, le glaucome néovasculaire.

14. Kit comprenant :
le composé destiné à être utilisé selon la revendication 1 ou 2, le produit destiné à être utilisé selon la revendication 6 où la composition pharmaceutique destinée à être utilisée selon la revendication 10 ; et
une instruction écrite décrivant un procédé pour administrer le produit pharmaceutique en combinaison avec un agent anti-VEGF pour prévenir ou traiter un trouble accompagné par une angiogenèse oculaire et/ou une perméabilité vasculaire oculaire accrue.
